# EUROPEAN PATENT APPLICATION

(11) **EP 0 712 828 A1**
(43) Date of publication of application: **22.05.1996**
(21) Application number: 95203083.1
(22) Date of filing: 13.11.1995
(51) Int. Cl.: C07C 45/50, C07F 9/6574, B01J 31/18

(54) **Process for preparing an aldehyde**

(30) Priority: 17.11.1994 EP 94203345
(71) Applicant: DSM N.V., NL-6411 TE Heerlen (NL); E.I. DU PONT DE NEMOURS AND COMPANY, Wilmington Delaware 19898 (US)
(72) Inventor: Hansen, Carolina B., NL-6136 BT Sittard (NL); Teunissen, Antonius J.J.M., NL-6165 XM Geleen (NL)
(74) Representative: Geerts, Johanna Adriana Maria

(57) **Abstract**

Process for preparing an aldehyde compound by hydroformylation of an ethylenically unsaturated organic compound wherein a catalyst system is used, which catalyst system comprises rhodium and a bidentate phosphite ligand, wherein the bidentate phosphite ligand is a compound according to formula (1), 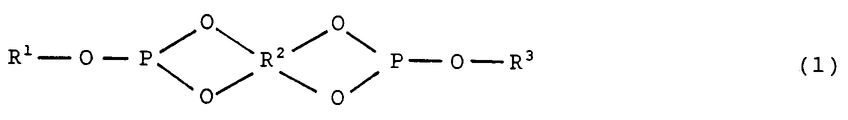
wherein R¹ and R³ are respectively an organic group which maybe the same or different and wherein R² is a tetravalent organic group.

## Description

The invention relates to a process for preparing an aldehyde compound by hydroformylation of an ethylenically unsaturated organic compound wherein a catalyst system is used which catalyst system comprises rhodium and a bidentate phosphite ligand.

With hydroformylation is meant the reaction of an ethylenically unsaturated organic compound with hydrogen and carbon monoxide in the presence of a hydroformylation catalyst.

A hydroformylation process for preparing aldehydes in which the catalyst system comprises rhodium and a bidentate phosphite ligand is for example described in EP-A-518241. In this patent application bidentate phosphite ligands according to the general formula A-[-O-P(OR)(OR)]₂ are described (A being a divalent organic group and R an organic group). The hydroformylation of for example 1-octene is described.

A disadvantage of the above hydroformylation process is that a large portion of the unsaturated organic compound is hydrogenated to the corresponding saturated organic compound, an unwanted byproduct.

The object of this invention is to provide a hydroformylation process with a higher selectivity to aldehydes and a lower degree of hydrogenation when compared to the process described in EP-A-518241.

The object of the invention is achieved in that the bidentate phosphite ligand is a compound according to formula (1), 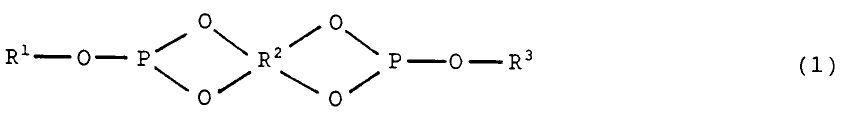
wherein R¹ and R³ are organic groups which may be the same or different and wherein R² is a tetravalent organic group.

It has been found that when preparing an aldehyde compound with the process according to the invention the selectivity to aldehydes is higher and the hydrogenation of the unsaturated starting compound is lower compared to the process described in EP-A-518241.

Some of the compounds that can be used as bidentate phosphite ligands according to the invention are described in US-A-5103035 in particular those in which R² is a tetra methyl methane group and R¹ and R³ are alkyl-substiuted phenyl groups. These phosphite compounds, are, according to US-A-5103035, used as stabilizers for polymer compositions. The description of US-A-5103035 does not suggest that these stabilizer compounds can be advantageously used as part of a hydroformylation catalyst.

R¹ and R³ may contain a heteroatom, for example N, S, Si or Sn. R¹ and R³ are preferably, the same or different, alkyl, cycloalkyl, aryl, aralkyl or alkaryl groups which groups have 1 to 30 carbon atoms and may be substituted with organic or inorganic groups. Examples of possible groups for R¹ and R³ are the tert-butyl, isopropyl, cyclopentyl, cyclohexyl, phenyl, naphtyl, benzyl, phenethyl, mesityl, tolyl and xylyl groups. Preferably R¹ and R³ are aryl, alkaryl or aralkyl groups for example phenyl, benzyl or naphtyl. More preferably R¹ and R³ are aryl groups.

R¹ and R³ may be substituted and examples of possible substituents are alkyl, aryl, alkoxy groups with 1 to 12 carbon atoms, trimethylsilyl groups with 1 to 12 carbon atoms and halogen groups, for example fluoride, chloride or bromide groups. When R¹ and/or R³ are substituted aryl or aralkyl groups, R¹ and/or R³ are preferably ortho substituted. R¹ and/or R³ are preferably ortho substituted with a substituent chosen from the group consisting of tert-butyl, tert-propyl, tert-amyl or trimethylsilyl.

R² is in general a tetravalent organic group with at least 4 carbon atoms. Generally the tetravalent organic group has less than 35 carbon atoms.

Other examples of possible bidentate phosphite ligands which can be used in the process according to the invention are the ligands according to formula (2)-(4) in which H₃₇C₁₈ represents a linear alkyl group with 18 carbon atoms, Me represents a methyl group and t.Bu represents a tert-butyl group: 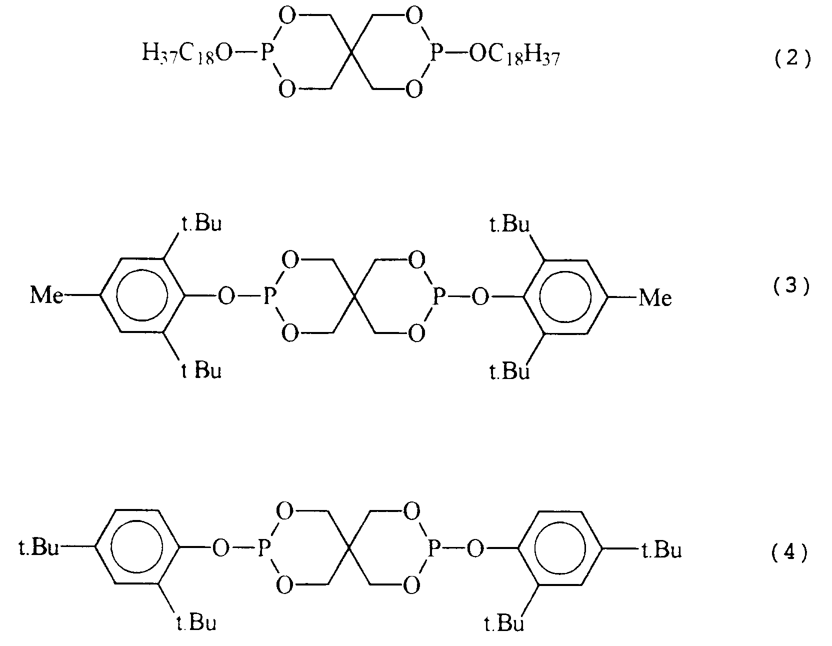
Preferably R² is a tetravalent organic group which group is flexible enough to allow both phosphorus atoms of the ligand to coordinate with one rhodium atom with formation of a chelate complex. By a chelate complex is meant that (substantially) the two phosphorus atoms of the bidentate phosphite ligand form a coordinated bond with one rhodium atom/ion. By a non-chelate-type complex is meant that substantially only one of the two phosphorus atoms of a bidentate phosphite ligand forms a coordinated bond with one rhodium atom or ion. The choice of the bridging group R² will determine wether a chelate complex of the bidentate phosphite ligand and rhodium is formed. Ligands with sufficiently flexible groups R² will form a chelate complex with rhodium. The ligands according to formula (2), (3) and (4) and the compounds as disclosed in US-A-5103035 do not posses a bridging group flexible enough to form a chelate complex.

It has been found that when bidentate ligands with such a flexible bridging group R² are part of the catalyst system the selectivity of the hydroformylation reaction is further improved especially when starting from terminally unsaturated organic compounds for example α-olefins.

The bridging group R² between the two phosphorus atoms preferably is flexible enough to allow both phosphorus atoms to coordinate with one rhodium atom ion. In order to determine whether a ligand is coordinated to a metal as a chelate complex, several techniques are available for the expert. Common techniques are X-ray crystallography on crystals of the rhodium complex, or NMR techniques such as ³¹P, ¹³C and ¹⁰³Rh NMR. The latter techniques are described in chapter 10 of "The organometallic chemistry of the transition metals" by R.H. Cabtree, John Wiley & Sons, New York, 1994.

Flexible bridging groups R² are preferably tetravalent organic groups comprising at least 8 carbon atoms for example aryl and alkyl groups. The four radical positions of the tetravalent organic group are the positions at which the four oxygen atoms of the bidentate phosphite ligand are bonded to the bridging group. Heteroatoms, for example N, S and O, may be present in the organic group R². Examples of possible flexible bridging groups R² are alkatetrayl groups, ortho-arylene -(Q)ₙ- ortho-arylene groups, alkylene -(Q)ₙ-alkylene groups or ortho-arylene -(Q)ₙ- alkylene groups, in which n = 0, 1, 2, 3 or 4 and Q is an arylene group, a -(CR⁴R⁵)- group in which R⁴ and R⁵ individually represent hydrogen or an alkyl group with one to 12 carbon atoms or Q is -O- (in which n = 1). The ortho-arylene group is preferably an ortho-phenylene group. Examples of possible alkylene groups, which are part of the tetravalent organic group as described above, are 1,3 isopropanediyl and 1,4-isobutanediyl. Examples of ortho-arylene groups, which are part of the tetravelent organic group as described above, are ortho-phenylene and ortho-naphthylene in which the (Q)ₙ group can be bound to the benzene ring at (any of) the numbered positions in formulas (5)-(7): 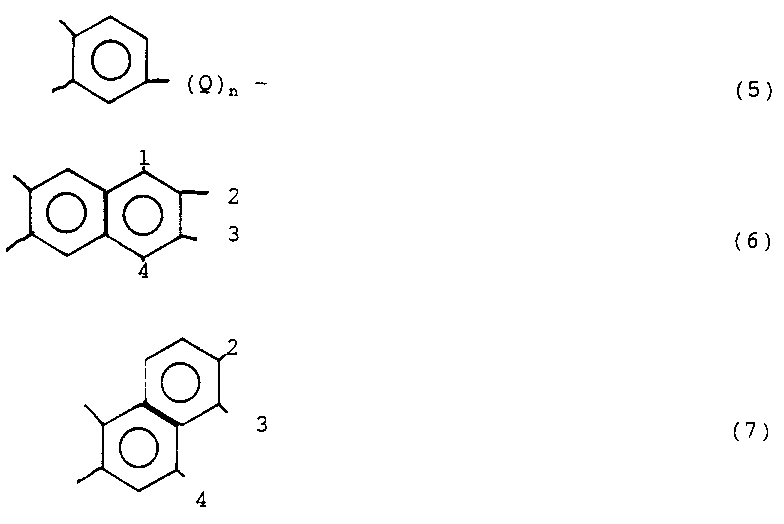
The tetravalent alkatetrayl group, R², in general will have 8 to 15 carbon atoms and can for example be represented by the following formula (8): 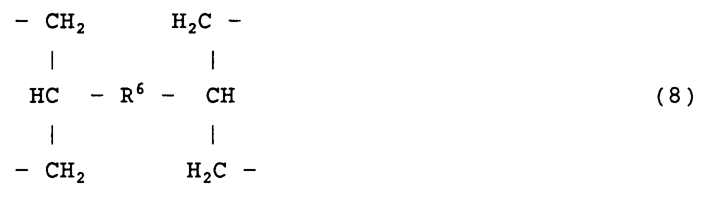
in which R⁶ represents an alkylene with at least 2 carbon atoms. Preferably R⁶ has 2 to 7 carbon atoms. Examples of possible groups R⁶ are ethylene, trimethylene, diethylene-ether, tetramethylene, pentamethylene and hexamethylene.

The ortho-arylene and alkylene as described above may be substituted with alkyl groups with 1 to 12 carbon atoms, phenyl, tolyl, anisyl or halogen groups, for example fluoride, chloride and bromide.

Examples of possible bidentate phosphite ligands with flexible bridging groups R² are represented by the formulas (9)-(12), in which MeO is a methoxy group and the "-C-C-C-" groups represent saturated divalent alkyl groups. 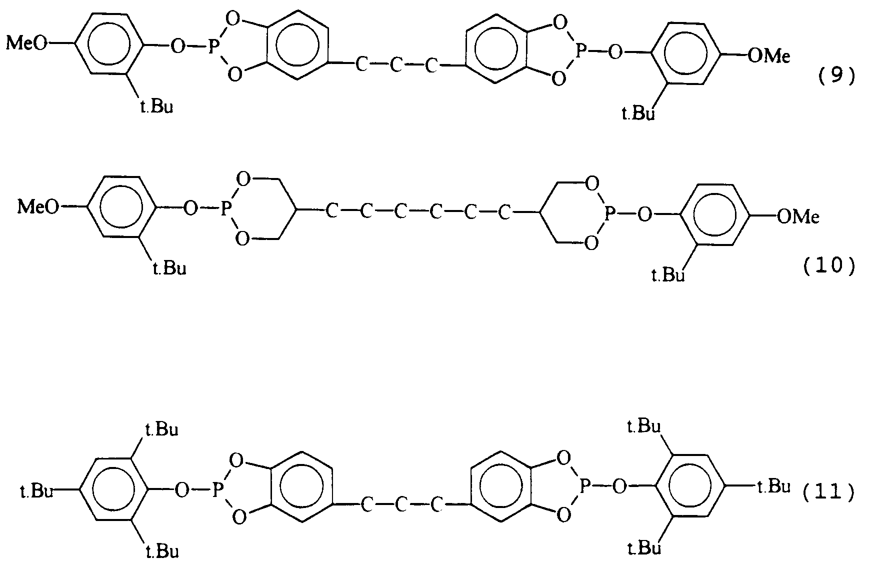 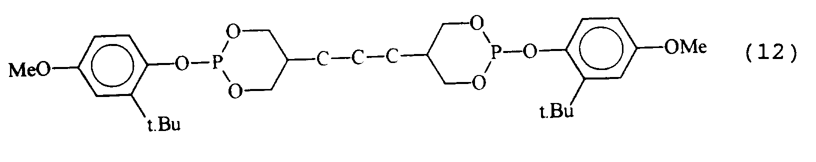
The bidentate phosphite compound according to formula (1) can for example be prepared in the same manner as described in the earlier mentioned US-A-5103035.

Generally the bidentate phosphite compounds according to formula (1) will be prepared starting from (a) a compound having four hydroxyl groups (R²(OH)₄), (b) a phosphorous compound and (c) a compound (or mixture of compounds) having one hydroxyl group (R¹OH and R³OH). Preferably the phosphorus compound is a phosphorous halogenide, for example PCl₃. Preferably the bidentate ligand is prepared by first preparing a alkyl or aryl phosphorouschloride by reaction of the starting phosphorus compound with the molecule having four hydroxyl groups functions. The alkyl or aryl phosphorouschloride is further reacted with the compound having one alcohol function in order to obtain the desired ligand. If necessary, the bidentate phosphite compound can be purified by crystallization or column chromatography.

The catalyst system can be prepared by mixing a suitable rhodium compound with the bidentate phosphite ligand in a suitable solvent in accordance with a well known complex-forming method. Suitable rhodium compounds are hydrides, halides, organic acid salts, inorganic acid salts, oxides, carbonyl compounds and amine compounds of rhodium.

Examples of suitable rhodium compounds are for example RhCl₃, Rh(NO₃)₃, Rh(OAc)₃, Rh₂O₃, Rh(acac)(CO)₂ [Rh(OAc)(COD)]₂, Rh₄(CO)₁₂, Rh₆(CO)₁₆, RhH(CO)(Ph₃P)₃, [Rh(OAc)(CO)₂]₂ and [RhCl(COD)]₂ (wherein "acac" is an acetylacetonate group; "Ac" is an acetyl group; "COD" is 1,5-cyclooctadiene; and "Ph" is a phenyl group). However, it should be noted that the rhodium compounds are not limited to the above listed compounds.

The molar ratio bidentate phosphite ligand and rhodium of the catalyst system is not specially limited, but is preferably selected so that favorable results are obtained in respect to catalyst activity and selectivity. This ratio generally is from about 1 to 100 and preferably from 1 to 25 (mol phosphite/mol metal).

The amount of rhodium used in the hydroformylation is not specially limited, but is preferably selected so that favourable results are obtained in respect of catalyst activity and economy. In general the amount of rhodium is between 10 ppm and 1000 ppm.

The choice of the reaction medium is not critical. The reaction medium may be the mixture of reactants of the hydroformylation itself, such as the unsaturated starting compound, the aldehyde product and/or by-products. If a solvent is used this may be for example an aromatic solvent, for example benzene, toluene, xylene, and dodecylbenzene; a ketone, for example acetone, diethylketone and methylethylketone; an ether, for example tetrahydrofuran and dioxane or an ester, for example ethylacetate and di-n-octylphthalate. A mixture of solvents may also be used.

The ethylenically unsaturated organic compound used in the preparation of an aldehyde compound is not specially limited so long as it has at least one ethylenically (C=C) bond in the molecule. The ethylenically unsaturated organic compound has usually 2 to 20 carbon atoms. Examples of possible ethylenically unsaturated organic compound are linear terminally olefinic hydrocarbons for example ethylene, propylene, 1-butene, 1,3-butadiene, 1-pentene, 1-hexene, 1-octene, 1-nonene, 1-decene, 1-tetradecene, 1-hexadecene, 1-octadecene, 1-eicosene and 1-dodecene; branched terminally olefinic hydrocarbons for example isobutene and 2-methyl-1-butene; linear internally olefinic hydrocarbons for example cis- and trans-2-butene, cis- and trans-2-hexene, cis- and -trans-3-hexene, cis- and trans-2-octene and cis- and trans-3-octene; branched internally olefinic hydrocarbons for example 2,3-dimethyl-2-butene, 2-methyl-2-butene and 2-methyl-2-pentene; terminally olefinic hydrocarbon-internal olefinic hydrocarbon mixtures for example octenes prepared by dimerization of butenes, olefin oligomer isomer mixtures (of from dimer to tetramer) of lower olefins including propylene, n-butene, isobutene or the like; and cycloaliphatic olefinic hydrocarbons for example cyclopentene, cyclohexene, 1-methylcyclohexene, cyclooctene and limonene.

Examples of the olefinic compound substituted with a hydrocarbon group containing an unsaturated hydrocarbon group include olefinic compounds containing an aromatic substituent, for example styrene, α-methylstyrene and allylbenzene; and diene compounds, for example 1,5-hexadiene, 1,7-octadiene and norbornadiene.

The ethylenically unsaturated organic compound can be substituted with one or more functional groups containing a heteroatom, for example oxygen, sulfur, nitrogen and phosphor. Examples of these substituted ethylenically unsaturated organic compound are vinyl methyl ether, methyl oleate, allyl alcohol, oleyl alcohol, 3-methyl-3-buten-1-ol, methyl 3-pentenoate, methyl 4-pentenoate, 3-pentenoic acid, 4-pentenoic acid, 3-hydroxy-1,7-octadiene, 1-hydroxy-2,7-octadiene, 1-methoxy-2,7-octadiene, 7-octen-1-al, hexa-1-en-4-ol, acrylonitrile, acrylic acid esters for example methylacrylate, methacrylic acid esters for example methylmethacrylate, vinyl acetate and 1-acetoxy-2,7-octadiene.

Preferred substrates are pentenenitrile, pentenoic acid and C₁-C₆ alkyl pentenoate ester compounds, for example 3-pentenenitrile, 3-pentenoic acid, methyl 3-pentenoate, ethyl 3-pentenoate and methyl 4-pentenoate. These compounds are preferred because the resulting aldehyde compounds can be advantageously used in the preparation of Nylon-6 and Nylon-6.6.

The reaction conditions to conduct the hydroformylation according to the invention, will be dependent of the particular starting ethylenically unsaturated organic compound. Generally the temperature can be from room temperature to 200 °C and preferably from 50 to 150 °C. The pressure is generally from (normal) atmospheric pressure to 20 MPa and preferably from 0.2 to 10 MPa and more preferably from 0.5 to 5 MPa. The pressure is generally the combined hydrogen and carbon monoxide partial pressure. Inert gasses may however be present. The molar ratio hydrogen : carbon monoxide is generally between 10:1 and 1:10 and preferably between 2:1 and 1:2.

Examples of the reaction system of the hydroformylation reaction include a continuous type, semicontinuous type or batch type operation using for example a stirring type tank reactor or a bubble tower type tank reactor.

The invention also relates to a new catalyst system comprising a bidentate phosphite ligand according to formula (1) and a metal of Group VIII of the Periodic System. The catalyst system can be used as a homogeneous catalyst for various reactions. The catalyst system can for example be used as a hydrocyanation, polymerisation, hydroformylation, isomerisation and carbonylation catalyst. It has been found that the above described bidentate phosphite ligand can advantagously be applied in combination with a Group VIII metal as a hydroformylation catalyst. The Group VIII metal is preferably rhodium when the catalyst system is used for hydroformylation. Examples of suitable Group VIII metals are cobalt, ruthenium, rhodium, palladium, platinum, osmium and iridium. Examples of Group VIII metal compounds include ruthenium compounds for example Ru₃(CO)₁₂, Ru(NO₃)₃, RuCl₃(Ph₃P)₃ and Ru(acac)₃; palladium compounds for example PdCl₂, Pd(OAc)₂, Pd(acac)₂, PdCl₂(COD) and PdCl₂(Ph₃P)₂; osmium compounds for example Os₃(CO)₁₂ and OsCl₃; iridium compounds for example Ir₄(CO)₁₂ and IrSO₄; platinum compounds for example K₂PtCl4, PtCl₂(PhCN)₂ and Na₂PtCl₆.6H₂O; cobalt compounds for example CoCl₂ Co(NO₃)₂, Co(OAc)₂ and Co₂(CO)₈; and rhodium compounds as described above.

A preferred catalyst system comprises a chelate-type complex of a bidentate phosphite ligand according to formula (1) and rhodium.

The invention also relates to the class of new phosphite compounds according to formula (1) in which the bridging group R² is flexible enough to allow both phosphorus atoms of the ligand to coordinate with one rhodium atom as also described above.

These new phosphite compounds can be prepared according to the above described processes.

Now, the present invention will be described in further detail with the reference to Examples. However, it should be understood that the present invention is by no means restricted to such specific Examples.

### Example I

The phosphite according to formula 9 was prepared by first preparing an aromatic molecule with four alcohol functions. This molecule was prepared by first reacting 3,4-dimethoxy acetophenone with 3,4-dimethoxybenzaldehyde according to the procedure described in Vogel "A text-book of practical organic chemistry", Longmans, 1966, page 718, for benzalacetophenone using 3,4-dimethoxyacetophenone instead of benzaldehyde. The thus obtained 3,4-dimethoxybenzal 3,4-dimethoxyacetophenone was hydrogenated using Pd/silica gel in ethylacetate/ethanol (3:1) as described in J. Agric. Chem. Soc. Jpn. 27 (1953) 491. Subsequently, the carbonyl function was reduced by a Clemmensen clevage. After clevage of the methoxy functions with HI the desired alcohol was obtained. The phosphite was prepared by reaction of 2-tert-butyl-4-methoxyphenyl phosphorus dichloride with the above prepared alcohol in toluene/dichloromethane. Purification was performed by column chromatography (Al₂O₃, CH₂Cl₂).

### Example II

The phosphite according to formula 10 was prepared by first preparing an aliphatic molecule with four alcohol functions, by reacting 1,6-dibromohexane with dimethylmalonate as described by G.R. Newkome et al in J. Am. Chem. Soc. 112 (1990) 8458. Subsequently, the ester groups in the resulting molecule were reduced with LiAlH₄ to obtain the alcohol. The phosphite was prepared as described in example I of US-A-5103035 in which 2-tert-butyl-4-methoxhyphenol instead of 2,4-di-tert-butylphenol and the above prepared alcohol was used. Purification was performed by column chromatography (Al₂O₃, CH₂Cl₂).

### Example III

The phosphite according to formula 11 was prepared using the method as described in Example I using 2,4,6-tri-tert-butylphenyl phosphorus dichloride instead of 2-tert-butyl-4-methoxyphenyl phosphorus dichloride.

### Example IV

The phosphite according to formula 12 was prepared using the method as described in Example II starting from 1,3-dibromopropane and dimethyl malonate.

### Example V

A Hastelloy-C-steel autoclave having an internal volume of 150 ml was filled in an atmosphere of nitrogen with 5.8 mg (2.25*10⁻⁵ mol) Rh(acac)(CO)₂, 7.88*10⁻⁵ mol phosphite according to formula 9 and 60 ml toluene. The autoclave was closed and flushed with nitrogen. During 1.5 hours the autoclave was heated to 90°C and the pressure was increased to 1.0 MPa with a H₂/CO mixture (1 : 1 (mol : mol)). Subsequently a mixture of 5.1 g (45 mmol) methyl 3-pentenoate and 1 g nonane (gas chromatography internal standard) and an amount of toluene resulting in a 15 ml mixture was injected. The reaction mixture was analyzed by gas chromatography after 1.5 hour. The results are presented in Table 1.

### Example VI

Example V was repeated with a phosphite ligand according to formula 10. The amount of rhodium was the same as in Example V. See Table 1 for the bidentate phosphite/Rh ratio, reaction time and results.

### Example VII

Example V was repeated with a phosphite ligand according to formula 11. See Table 1 for results.

### Example VIIIa and VIIIb

Example V was repeated with a phosphite ligand according to formula 12. See Table 1 for results.

### Example IX

Example V was repeated with phosphite ligand according to formula 3. The results are presented in Table 1.

### Example X

Example V was repeated with phosphite ligand according to formula 4. The results are presented in Table 1.

### Comparative Example A

Example VIIIb was repeated with an equimolar amount of the following phosphite ligand (formula 35 of EP-A-518241): 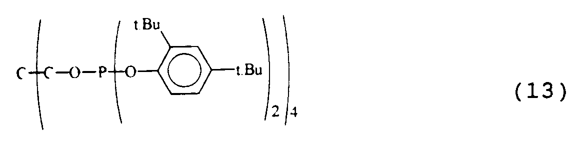
See Table 1 for results.

### Comparative Example B

Example VIII was repeated with an equimolar amount of the following bidentate phosphite ligand: 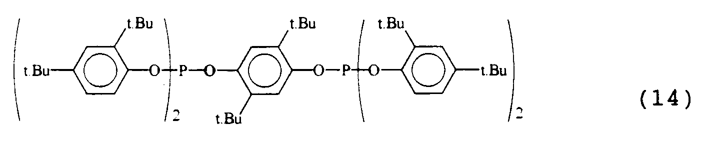
See Table 1 for results.

**Table 1**

| Example | phosphite ligand (L) | L/Rh ⁽¹⁾ | time (hours) | conversion (mol%) | selectivity to aldehydes (mol%)⁽²⁾ | hydrogenation (mol%) |
|---|---|---|---|---|---|---|
| V | 9 | 3.5 | 1.5 | 27.4 | 82.7 | 17.3 |
| VI | 10 | 1.0 | 2.5 | 44.5 | 94.5 | 5.5 |
| VII | 11 | 7.8 | 24 | 10.7 | 83.9 | 16.1 |
| VIIIa | 12 | 25 | 24 | 68.7 | 90.4 | 9.6 |
| VIIIb | 12 | 5 | 25 | 98.3 | 87.0 | 13.0 |
| IX | 3 | 2 | 20.5 | 68.2 | 97.5 | 1 |
| X | 4 | 2 | 4 | 56.4 | 99.0 | 2.5 |
| A | 13 | 5 | 16 | 92.4 | 80.3 | 19.7 |
| B | 14 | 5 | 16 | 88.8 | 83.6 | 16.4 |

| | | | | | | |
|---|---|---|---|---|---|---|
| ⁽¹⁾ L/Rh ligand/Rh ratio in mol/mol | | | | | | |
| ⁽²⁾ Selectivity is mol aldehydes formed/mol substrate converted * 100% | | | | | | |

The results in Table 1 illustrate the lower rate of hydrogenation and the higher selectivity to aldehyeds when the process according to the invention is used compared to when the process according to the state of the art is used as exemplified in Experiments A and B.

### Example XI

Example V was repeated with the same amount of rhodium and a rhodium/bidentate phosphite ratio of 2. Instead of methyl 3-pentenoate trans-2-octene was used as substrate. The results are presented in Table 2.

### Comparative Example C

Example XI was repeated with a phosphite according to formula 14. See Table 2 for results.

**Table 2**

| Example | ligand (L) | time (hours) | conversion (mol%) | selectivity to aldehydes (mol%) | hydrogenation (mol%) |
|---|---|---|---|---|---|
| XI | 10 | 20 | 30.7 | 99.2 | 0.8 |
| C | 14 | 94 | 49.3 | 98.9 | 1.1 |

### Example XII

Example V was repeated in which a phosphite according to formula 10 with a L/Rh = 2 and 1-octene as substrate is used. The results are presented in Table 3.

### Example XIII

Example XII was repeated using a phosphite according to formula 3. The results are presented in Table 3.

### Comparative Example D

Example XII was repeated with a phosphite according to formula 14. The results are presented in Table 3.

**Table 3**

| Example | ligand (L) | time (hours) | conversion (mol%) | selectivity to aldehydes (mol%) | hydrogenation (mol%) |
|---|---|---|---|---|---|
| XII | 10 | 3 | 93.0 | 99.7 | 0.3 |
| XIII | 3 | 3 | 80.6 | 98.9 | 1.1 |
| D | 14 | 3 | 57.9 | 97.7 | 2.3 |

Table 3 illustrates for instance that when a chelate comples is used as in Example XII higher the conversion and selectivity to aldehydes and a lower rate of hydrogenation is achieved than when non-chelate complex is used (Example XIII).

## Claims

1. Process for preparing an aldehyde compound by hydroformylation of an ethylenically unsaturated organic compound wherein a catalyst system is used, which catalyst system comprises rhodium and a bidentate phosphite ligand, characterized in that the bidentate phosphite ligand is a compound according to formula (1), 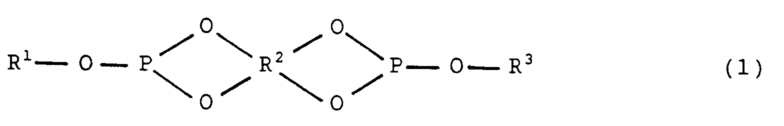 wherein R¹ and R³ are organic groups which may be the same or different and wherein R² is a tetra valent organic group.

2. Process for preparing an aldehyde according to claim 1, characterized in that R² is a group which is flexible enough to allow both phosphorus atoms of the phosphite ligand to coordinate with one rhodium atom.

3. Process according to claim 2, characterized in that R² is a tetravalent organic group comprising at least 8 carbon atoms.

4. Process according to claim 3, characterized in that R² is a alkatetrayl group, an ortho-arylene-(Q)ₙ-ortho-arylene group, an alkylene -(Q)ₙ-alkylene group or an ortho-arylene -(Q)ₙ-alkylene group, in which n = 0, 1, 2, 3 or 4 and Q is a divalent aryl group or alkyl group.

5. Process according to claim 4, characterized in that R² is an ortho-phenylene-(Q)ₙ-ortho-phenylene group and Q is an -(CR⁴R⁵)- group in which R⁴ and R⁵ individually represent hydrogen or an alkyl group with 1 to 12 carbon atoms.

6. Process according to claim 4, characterized in that R² is represented by the following formula: 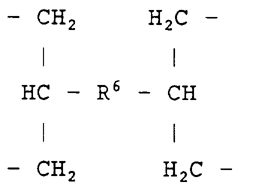 in which R⁶ is a alkylene group with 2-7 carbon atoms.

7. Process according to any one of claims 1-6, characterized in that the ethylenically unsaturated organic compound is a pentenenitrile, pentenoic acid or a C₁-C₆ alkyl pentenoate ester.

8. Catalyst system comprising a bidentate phosphite ligand as described in any one of claims 1-6 and a Group VIII metal.

9. Catalyst system according to claim 8, characterized in that the Group VIII metal is rhodium.

10. Phosphite compound according to formula (1) in which the bridging group R² is flexible enough to allow both phosphorus atoms of the compound to coordinate with one rhodium atom.

11. Process for preparing a phosphite compound according to claim 10, in which a compound corresponding with R² having four alcohol functions is reacted with a phosphoroushalogenide compound and the obtained organic phosphoroushalogenide compound is then reacted with a mono-alcohol compound corresponding with R¹OH or R²OH.
